(19) Europäisches Patentamt
European Patent Office
Office européen des brevets

(11) Publication number: **0 501 048 A1**

## (12) EUROPEAN PATENT APPLICATION

(21) Application number: **91301484.1**

(22) Date of filing: **25.02.91**

(51) Int. Cl.⁵: **A61N 2/02**, A61N 1/40

(43) Date of publication of application:
**02.09.92 Bulletin 92/36**

(84) Designated Contracting States:
**AT BE CH DE DK ES FR GB GR IT LI LU NL SE**

(71) Applicant: **LTI-IMD USA, INC.**
**1637, Grant Road,**
**Mountain View, California 94040(US)**

(72) Inventor: **Griffith, Neil J.**
**3959 Sequoia Street**
**San Diego California 92109(US)**

(74) Representative: **Wilson, Nicholas Martin et al**
**WITHERS & ROGERS 4 Dyer's Buildings**
**Holborn**
**London EC1N 2JT(GB)**

(54) Shielded electromagnetic transducer.

(57) An electrical to electromagnetic transducer for applying electromagnetic energy to damaged potions of the living body, which provides high efficiency generation of electromagnetic fields for electromagnetic therapy by directing electromagnetic energy to the damaged body part. Electromagnetic energy is initially generated by a dipole (10) consisting of a bar (12) of high permeability material wrapped with an electrically conductive coil (16). The dipole (10) is placed between a conductive shield (15) and the damaged body part. An electrical signal passes through the coil (16) which causes a magnetic field to be generated through and around the high permeability material (12). The field radiation pattern of the dipole (10) is directed toward the damaged body part by a conductive shield (15). Magnetic fields which are generated away from the damaged body part intersect the conductive shield (15) and establish eddy currents. These eddy currents in turn generate magnetic fields opposite and nearly equal to the magnetic fields generated by the electromagnetic source. These resultant redirected electromagnetic fields then reinforce the electromagnetic field directed towards the damaged body part and diminish the electromagnetic field directed away from the damaged body part.

FIG. 2

**FIELD OF THE INVENTION**

The present invention relates to the field of transducers for converting electrical energy into electromagnetic fields. More specifically, the present invention relates to the use of transducers to provide electromagnetic fields to damaged body components to promote healing.

**BACKGROUND OF THE INVENTION**

The use of electric or electromagnetic fields to promote healing, particularly the healing of fractured bones, has been investigated since the early 19th Century. See Spadaro, "Bioelectric Stimulation of Bone Formation: Methods, Models and Mechanisms," *J. Bioelectricity, 1*, 1, 99-128 (1982). Initially, direct current techniques were used by applying electrodes to the skin or via the use of implanted electrodes into the bone. More recently, mechanisms which encourage growth have been investigated which involve the use of electromagnetic fields to induce voltage and current effects within the tissue. These techniques have been particularly useful in non-healing or "non-union" fractures by inducing bones to heal which will not heal naturally.

An example of a technique for the use of electromagnetic radiation to promote bone growth is disclosed in U.S. Patent No. 4,266,532. However, the techniques disclosed therein required the use of power applied from a standard wall socket. Electromagnetic therapy is only useful so long as the patient uses it. Being tethered to the wall is a sufficient annoyance such that many patients will not follow the electrotherapy regimen prescribed by their doctors.

In view of this problem, a great deal of work has occurred to try to develop mechanisms whereby portable electrotherapy may be provided. Examples of such techniques are shown in U.S. Patents Nos. 4,432,361; 4,574,809; and 4,587,957. The system shown in the first of these patents requires the use of invasive electrical probes into the bone. It is desirable to avoid invasive techniques if possible because of the possibility of infection. The systems of the other two patents use magnetic transducers and/or Helmholtz coils designed to provide a uniform electromagnetic field throughout the treatment area. The system of the third patent also included a polarized magnetic field. These types of transducers establish electromagnetic fields outside of the damaged body portion and waste a great deal of energy inside the damaged body portion by radiating areas where stimulation is unnecessary. For example, the tibia and scaphoid bones are very close to the surface of the skin. Use of the techniques shown in the above references causes radiation to be generated throughout the entire limb even though the damaged portion is close to the surface of the skin. This problem is even more striking in the case of bones in the trunk of the body such as vertebrae and ribs. Therefore, a technique for applying electromagnetic fields in a manner which concentrates their application on the desired area while minimizing wasted electromagnetic energy would be desirable.

The benefits of electromagnetic stimulation of damaged or diseased tissue are being further developed and are widely accepted by today's scientific community. Examples of studies which show the benefits of electromagnetic stimulation to soft tissue as well as bone are Black, "Electrical Stimulation of Hard and Soft Tissues in Animal Models," *Clinics in Plastic Surgery, 12*, 2 (April, 1985) and Frank et al., "A Review of Electromagnetically Enhanced Soft Tissue Healing," *IEEE Engineering in Medicine and Biology*, 27-32 (December, 1983). In addition, diseased rather than broken bones may benefit from electromagnetic therapy. For example, see U.S. Patent No. 4,467,808, and Bassett et al., "Treatment of Osteonecrosis of the Hip with Specific Pulsed Electromagnetic Fields (PEMFs): A Preliminary Clinical Report," *Bone Circulation*, Ch. 56, pp. 343-354, Arlet et al., eds. (1984).

**SUMMARY OF THE INVENTION**

The present invention provides an electrical to electromagnetic transducer for applying electromagnetic energy to damaged portions of the living body, to stimulate healing and help provide quicker recovery. The embodiments of the present invention provide high efficiency generation of electromagnetic waves for electromagnetic therapy by directing electromagnetic radiation to the damaged body part. In one embodiment, the electromagnetic radiation is initially generated by a dipole consisting of a bar of high permeability material wrapped with an electrically conductive coil. An electrical signal passes through the coil which causes a magnetic field to be generated through the high permeability material. The field pattern of the dipole is directed towards the damaged body part by a conductive shield. The electromagnetic field generator is placed between the conductive shield and the damaged body part. Magnetic fields which are generated away from the damaged body part intersect with the conductive shield. The change in the magnetic field establishes eddy currents within the conductive shield. These eddy currents generate magnetic fields opposite and nearly equal to the magnetic fields generated by the electromagnetic source. The magnetic fields generated by the eddy currents provide an electromagnetic field which reinforces fields directed towards the body part and diminish electromagnetic fields directed away from the damaged body part.

In another embodiment of the invention, the electromagnetic generator is a bar type dipole, arcuate dipole or compound (such as quadripole). Multiple separate electromagnetic generators are employed in still other embodiments, as well as variations on the spacing of the turns of the conductive coils.

In another embodiment of the present invention, a second piece of high permeability material is placed adjacent to the damaged body part but separated (e.g. diametrically opposed) from the shielded electromagnetic transducer. The presence of the high permeability material creates high electromagnetic flux lines directed towards the high permeability material. The high permeability material behaves as a magnetic conductor much like an electrical conductor alters an electric field. Thus, even more precise direction of the electromagnetic field may be achieved.

By directing the electromagnetic field only to the appropriate damaged body part, large savings in power dissipation are achieved. In addition, the deleterious effects of electromagnetic radiation on healthy tissue are minimized by precise direction of the electromagnetic field.

## BRIEF DESCRIPTION OF THE DRAWINGS

Figure 1 is a schematic diagram showing the flux lines emanating from a prior art dipole electromagnet;

Figure 2 is a schematic diagram showing the flux lines generated by a dipole transducer in conjunction with a conductive shield;

Figure 3 shows the application of shielded dipole transducer 10 to provide electromagnetic therapy to a broken tibia;

Figure 4 is a perspective view of the shielded dipole transducer shown in Figure 2;

Figure 5 is cross-sectional view of shielded dipole transducer 10 taken along cross-section line 4 of Figure 4;

Figure 6 is another cross-sectional view of shielded dipole 10 taken along section lines 5 of Figure 4;

Figure 7 shows the flux lines generated within the patient's leg by electrical, signals applied to the shielded dipole transducer;

Figure 8 is a diagram showing the wasted electromagnetic energy generated by an oversized shielded dipole transducer;

Figure 9 is a computer generated diagram showing the flux lines of a shielded dipole transducer as calculated by a computer simulation;

Figures 10A and 10B are graphs of representative signals applied to leads 17 of transducer 10;

Figure 11 is a chart showing the stiffness ratios of healed rabbit bones versus the applied search coil voltage;

Figure 12A is a graph showing the relationship between the intensity of the electromagnetic field and the stiffness of the bone treated using electromagnetic therapy;

Figure 12B is a graph showing the intensity of the electromagnetic field generated by a shielded dipole transducer as determined from the computer simulation shown in Figure 9;

Figure 13A is a schematic diagram showing an approximate equivalent circuit for shielded dipole 10;

Figure 13B is a schematic diagram reducing the circuit shown in Figure 13A to basic circuit elements;

Figure 14 is a side view diagram of another shielded dipole transducer 100 showing the altered flux lines by the modification of shielded dipole 100 as compared to shielded dipole 10;

Figure 15 is a side view diagram describing the alteration and the flux lines caused by altering the turn spacing of the coil of shielded dipole 110;

Figure 16A is an end view diagram showing the positioning of interlocking shielded dipole transducers around invasive bone stabilization equipment (e.g. external fixators) for providing electromagnetic therapy in conjunction with invasive technologies to treat extremely severe broken bones;

Figure 16B is a top view showing the interlocking shielding of the shielded dipole transistors 122 and 124 of Figure 16A;

Figure 17A is an end view diagram showing the positioning of an arcuate shielded dipole transducer on a leg;

Figure 17B is a side view diagram showing arcuate transducer 140 positioned on a leg;

Figure 18A is a top view diagram showing the positioning of a partially arcuate shielded dipole transducer;

Figure 188 is a side view diagram of transducer 150 of Figure 18A;

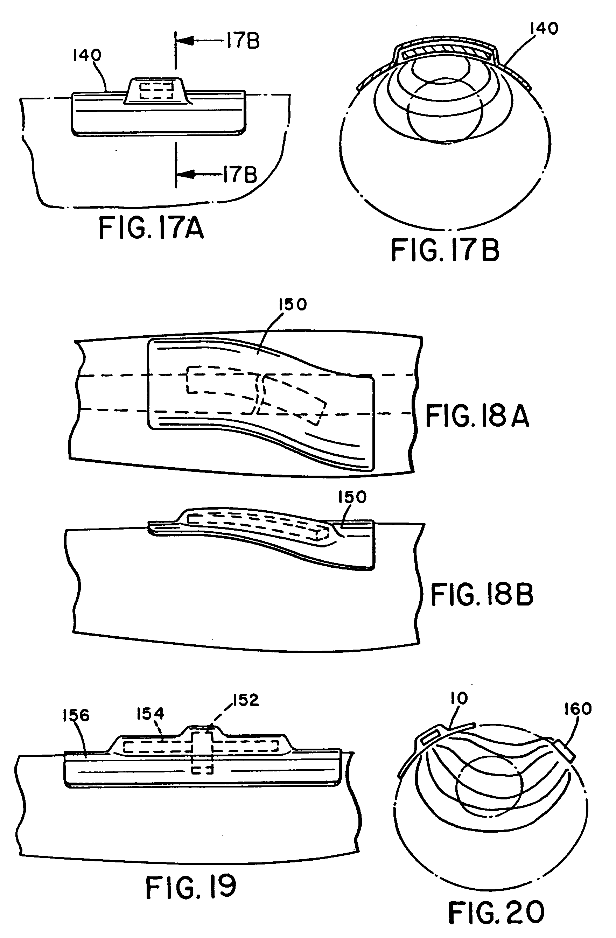

Figure 19 is a side view diagram showing a quadripole shielded transducer; and

Figure 20 is an end view diagram showing the lines of flux for positioning a portion of high permeability material in the vicinity of the operation of shielded dipole 10.

## DETAILED DESCRIPTION

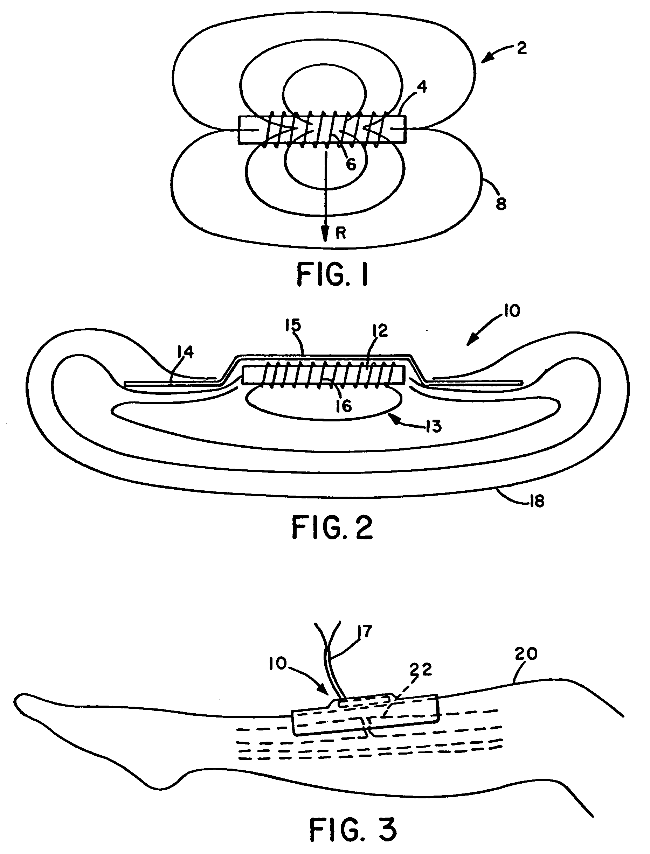

Figure 1 is a schematic diagram showing a prior art dipole electromagnet 2. High permeability bar 4 is wrapped by wire 6 a selected number of turns N. When current is passed through windings 6, a magnetic field is generated symbolized by flux lines 8. The intensity of the far magnetic field in a direction R perpendicular to dipole 2 is given [see Plonus, *Applied Electromagnetics*, 328 (1978)] by the equation:

$$B = \mu_0 nIA/4\pi R^3$$

where B is the absolute value of the intensity of the field,

$\mu_0$ is the permeability of air,

n is the number of turns of winding 6,

I is the current passing through winding 6,

A is the cross-sectional area of bar 4, and

R is the distance from the geometric center of bar 4.

A primary goal of the present invention is to efficiently apply electromagnetic energy to a damaged body portion. One major source of waste in the generation of electromagnetic fields is the generation of electromagnetic fields in areas where they are not useful. As can be seen from Figure 1, an equal amount of the electromagnetic field is generated above and below dipole 2. In fact, the electromagnetic lines of flux forms a toroidal shape centered around the major axis of bar 4.

Figure 2 is a side view diagram of one embodiment of the present invention showing a shielded dipole arrangement 10. Windings 16 are wrapped around high permeability bar 12 and current is passed through winding 16 to generate a magnetic field represented by flux lines 18. Dipole assembly 13 is positioned inside indentation 15 in conductive plate 14. Plate 14 can be formed of a number of known conductive materials. It is advantageous that the material used to construct conductive plate 14 is as flexible as possible and is as conductive as possible. I anticipate that an ideal conductive plate 14 will be a superconductor material.

When time varying current passes through winding 16, a time varying magnetic flux is generated. The magnetic flux which attempts to pass through conductive plate 14 induces eddy currents. These eddy currents generate magnetic flux which tends to oppose the magnetic flux which caused the eddy currents in the first place. This principle is known as Lenz' Law. If conductive plate 14 were a perfect conductor, the magnetic flux generated by the eddy currents would be opposite and equal to the magnetic flux which caused the eddy currents in the first place. Thus, the magnetic flux on the upper side of conductive plate 14 would be completely cancelled and the magnetic flux on the lower side of conductive plate 14 would be reinforced. However, ohmic losses in conductive plate 14 reduce the efficiency so that a partial magnetic field is generated above conductive plate 14. However, the eddy current induced magnetic flux provides a focusing action directing the magnetic field to one side and allows a concentration of the generated magnetic field into a selected body part to aid in the healing process.

Figure 3 is a side view diagram showing the placement of shielded dipole transducer 10 on a person's leg 20 to promote the healing of a broken tibia 22. Leads 17 indicate the connection to an electrical source for generating the magnetic field. Experiments have shown that an electrical signal comprised of a train of bursted symmetrical pulses having a frequency of up to 1 MHz in burst intervals of 5 msec provides optimal power efficiency with effective therapeutic benefit. This signal is provided to leads 17.

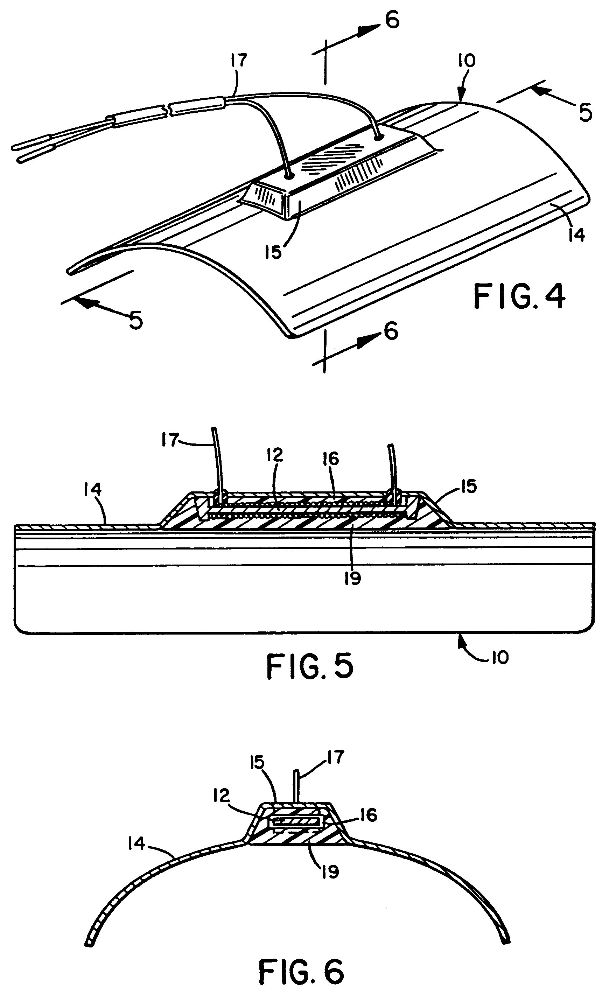

Figure 4 is a perspective view showing the shape of shielded dipole transducer 10.

Figure 5 is a cut-away portion along lines 5-5 of Figure 4 showing the construction of shielded dipole transducer 10. High permeability bar 12 and winding 16 are placed in indentation 15 and indentation 15 is filled with an epoxy material to 19 securely affix bar 12 and winding 16 in indentation 15. Leads 17 are brought out through the surface of field 14 for connection to a power source. Figure 6 is a cut-away view of transducer 10 on lines 6-6 of Figure 4.

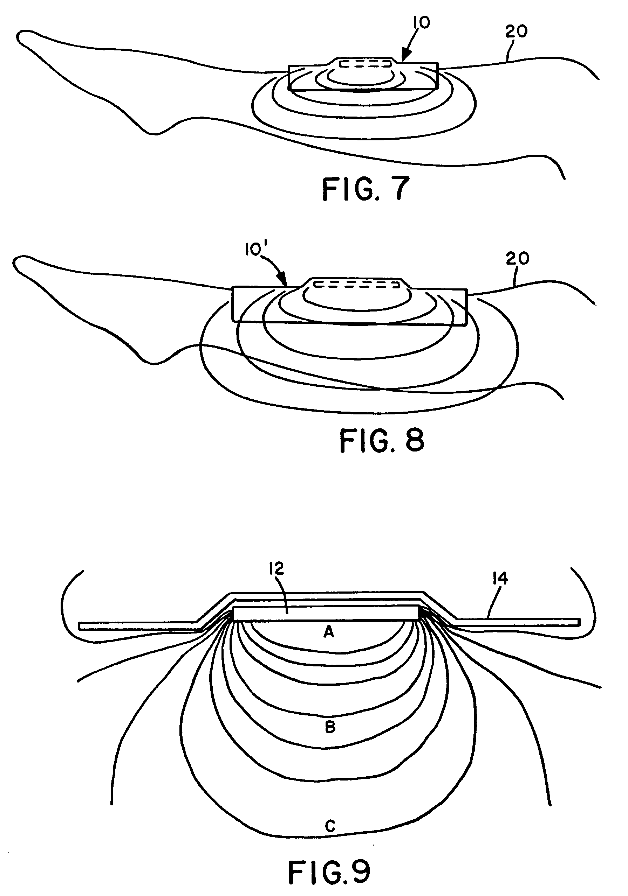

Figure 7 is a side view diagram showing how the electromagnetic field focusing aspects of shielded dipole 10 direct the electromagnetic field to the damaged body portion and maximize the efficiency of the applied energy.

It is important that the size of the shielded dipole transducer be appropriately selected for the depth of penetration required to induce healing in the appropriate body part.

As illustrated in Figure 8, an oversized shielded dipole will generate considerable electromagnetic radiation outside of leg 20 thereby wasting energy. The determination of the intensity of the electromagnetic field of a field generated by a shielded dipole transducer is extremely complex.

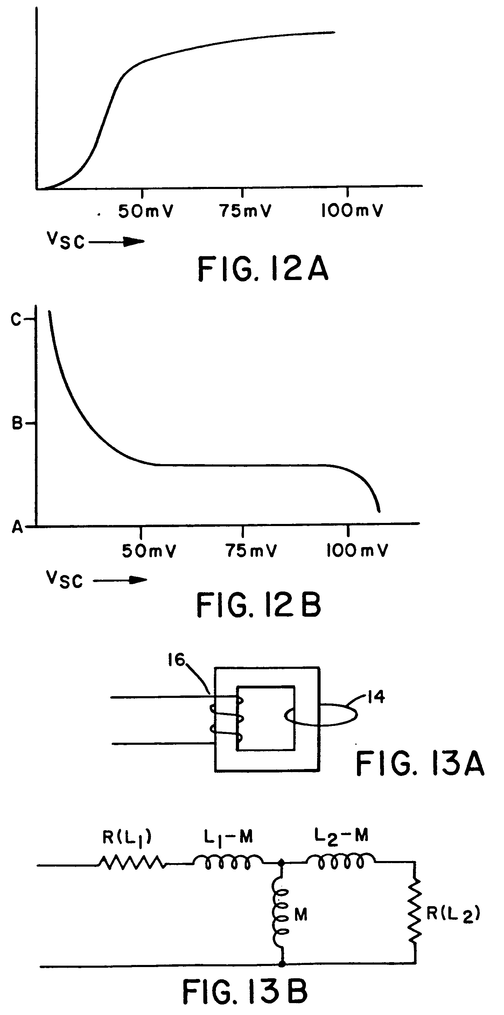

Figure 9 is a drawing taken from a computer generated diagram for synthesizing the electromagnetic field generated by a shielded dipole such as transducer 10. In this model the shield 14 is assumed by the computer to be an ideal conductor. Three points, A, B and C (Figure 12B) are indicated on the diagram. A indicates the intensity of the electromagnetic field directly beneath shielded dipole 10. B indicates the level where the electromagnetic field corresponds to the minimum effective field for promoting healing. C is an arbitrarily chosen far field point.

Portability is preferably realized by integrating or associating the transducer with the cast around the fracture site, and having the power (battery) source and signal generator located a short distance away. For example, the latter components might be attached to the user's waist belt and connected via leads to the transducer. Alternately, all the components may be attached to or integrated into the cast. The former situation may be favored when a patient needs constant electrostimulation which may necessitate frequent replacement of batteries. On the other hand, for patients with simple or small geometry fractures, the time of application needed for maximum rate of healing may be considerably reduced, and there may be no need to change batteries over the required stimulation period. Here it might be desirable, for the patient's convenience and aesthetic purposes, to integrate the entire unit into the cast. Any apparatus installation which minimizes patient involvement or apparatus obtrusiveness, greatly enhances the likelihood of success through improved patient compliance.

It is anticipated that the power source and signal generator circuitry used to generate the therapeutic signal will weigh less than 1 pound and be about the size of a common pocket camera. Typically the battery voltage will be on the order of 6-40 V and the unit will accommodate a battery having a volume on the order of about 2-60 $cm^3$. It has been determined that higher voltages are more efficient than lower voltages. However, 40 V is the generally accepted maximum voltage that can be applied to humans without substantial injury. Present devices operate at 35 V to allow a 5 V margin in case of accidental application of the driving voltage to the patient.

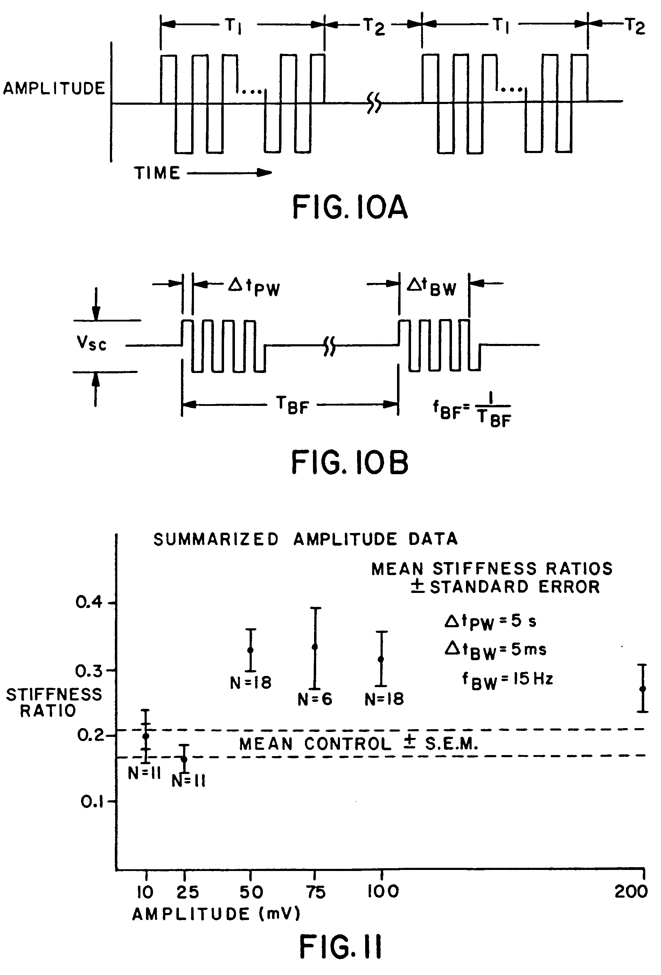

The signal applied to leads 17 is illustrated in Figures 10A and 10B. The therapeutic effects of the signal of Figures 10A and 10B were established using an animal model system. In the signal shown in Figures 10A and 10B $V_{sc}$ is the search coil voltage; $\Delta t_{PW}$ is pulse width; $\Delta t_{BW}$ is burst width and $f_{BF}$ is burst frequency. The search coil voltage is that voltage which is generated by a time varying magnetic field through a single loop of conductor having an internal area of 1 $cm^2$.

A $\Delta t_{PW}$ in the range of 0.5-20 $\mu$sec, preferably 2-10 $\mu$sec, is therapeutically effective, with 5 $\mu$sec being particularly effective. While I do not wish to be bound to any specific theory of operation, I believe that the effectiveness of this invention conforms to theoretical considerations. Activation of the cellular machinery involved in bone repair by electromagnetic radiation requires delivery of a signal to the injured site having defined time constants for burst width and burst frequency. In order to realize this, it is necessary for the signal to be established in healthy tissue and to reach the injured site without being significantly attenuated by the tissue. This in turn suggests that the time constants associated with the magnetic, electric, chemical and electrodiffusion effects caused by the signal exhibit particular time constants. I believe that the magnetic "diffusion" equation assures that below 100 MHz the magnetic field completely penetrates through to the injured site. For electric "diffusion", penetration of the bone by the displacement current density remains low until 1 MHz (equivalent to $\Delta t_{PW}$ = 0.5 $\mu$sec). Further, the viscous flow of interstitial fluids in the canaliculi can follow frequencies up to 1 MHz. In contrast, however, mechanical stress frequency responses attenuate after 500 Hz. Based on this brief analysis, it is apparent that signal current densities with pulses widths as low as 0.5 $\mu$sec would likely be established in the tissue, and thus potentially produce therapeutically effective results.

I have found that the procedure is particularly effective when $V_{sc}$ is 74 mV, $\Delta t_{PW}$ is 5 $\mu$sec, $f_B$ is 15 Hz, $\Delta t_{BW}$ is 5 msec and $t_{PW}$ is varied from 2-10 $\mu$sec. Moreover, Figure 11 illustrates that the effectiveness of these parameters is a function of the amplitude of the signal. A search-coil voltage amplitude A of 25<A<200 mV is effective with 50-100 mV being particularly effective.

It will, of course, be appreciated that what has been described is a method for treating certain types of bone fractures and is believed applicable both to animals and to humans, and to stimulation of healing of various types of bone fractures and damaged tissues, including fresh fractures, and especially bone fractures that do not readily heal in the absence of treatment, such as delayed unions, non-unions and failed fusions.

Figure 12A is a graph depicting relative stiffness of osteotomized rabbit bones corresponding to the search coil current used to promote healing in the broken rabbit bones, and shows that both the minimum search coil voltage typically necessary for significant healing enhancement, although a broad range of search coil voltages provides effective therapy. Thus, uniformity of the applied field is not necessary for effective therapy and in fact, because the optimal voltage for each type of cell may vary, a range of applied signals increases the probability that the optimal voltage will stimulate these various cell types and thus enhance the healing process over the prior art teaching of uniform applied fields.

Figure 12B is a graph depicting the magnetic field generated by the simulation of Figure 9 in terms of search coil voltage. As can be seen from the graph of Figure 12B, point 8 is the farthest point from the shielded dipole which will provide adequate healing electromagnetic fields and that closer points to the shield have a signal which will provide effective therapy. For optimal efficiency, the size of shielded dipole 10 (Figure 7) should be chosen so that point B is just beyond the damaged body portion in which healing is to be promoted. The Table below shows the size of bar 12 (Figure 2) and the corresponding depth of penetration (DOP) and optimal shield length and width (both in cm).

TABLE

| TYPE | BAR LENGTH | DOP (cm) | SHIELD LENGTH | SHIELD WIDTH |
|------|-----------|----------|---------------|--------------|
| I | 4 | 2 | 8 | 6 |
| II | 8 | 4 | 16 | 12 |
| III | 12 | 6 | 24 | 18 |

As stated earlier, for maximum utility, the shield 14 should be flexible to allow the molding of the shield to conform to the damaged limb or other damaged body part. However, a conflicting goal is the requirement of maximum conductivity. An extremely thick plate is highly conductive but very inflexible. On the other hand, a thin plate is highly flexible but not very conductive. To determine an adequate compromise, the conductivity of the shield material at operating frequency must be determined. In direct current situations, the conductivity of the shield material is determined by the cross-sectional area of the conductor perpendicular to the direction of current flow. However, for efficient electromagnetic wave generations the higher the frequency generated, the greater the transfer efficiency into the electromagnetic field. Given a bursted rectangular waveform applied to leads 17 having a pulse width of approximately 5 $\mu$sec, the effective frequency F is $1/(2 \times 5 \mu s) = 100$ kHz. To obtain the Lenz effect for the ninth harmonic, and thus for approximately 90% of the signal, a thickness for the shield must be chosen so that the skin depth at this frequency is about one third of the thickness of the shield. One third of the thickness is for one surface of the shield, another is for the other surface and the third is for additional margin. The skin depth is determined by the equation:

$$D = (FMS) - 0.5$$

where
D is the skin depth,
F is the frequency of the signal,
M is the relative permeability, and
S is the shield material conductivity.

Solving these equations for copper and 900 kHz yields D(Cu) = 0.06 mm and thus a shield thickness of 0.2 mm.

The conductive material is preferably a highly conductive material such as copper or aluminum. Aluminum is also advantageous because it is X-ray transmissive. Thus adequate X-rays may be taken through the aluminum shield. Various configurations of shield material have proven efficacious. For example, a wire mesh and a metal foil affixed to a cloth backing has been used. A particularly advantageous embodiment is foil mounted on a cloth backing where a regular pattern of holes, such as diamond shaped holes, are formed in the foil. The holes allow lateral compression of the foil which allows excellent three dimensional conformability to the surface of the injured body portion.

The electrical characteristics of transducer 10 can be expressed as a loosely coupled transformer which is schematically represented by Figure 13A. Using the T network model for representation of transformers, an equivalent circuit can be shown as in Figure 13B. $R(L_1)$ is the resistance and $L_1$ is the inductance of coil 16. M is the transinductance or coupling of the "transformer." $R(L_2)$ is the resistance and $L_2$ is the inductance of shield 14. Treating the dipole itself as a separate inductor, and assuming that the solenoid has a length to diameter ratio of 1, the inductance is given by:

6

$$L \text{ (in } \mu\text{H)} = 6.8 \text{ Sn}^2$$

where

L is the inductance;

S is the diameter of the solenoid; and

n is the number of turns.

Different solenoid configurations require different analysis. Inductive analysis is well known in the art.

The ohmic energy losses are represented by the ohmic losses passing through $R(L_1)$ and $R(L_2)$. The resistance of the solenoid $R(L_1)$ is determined by taking the unit length resistance of the coil times the total length of the coiled wire. Because shield 14 is loosely coupled, M is nearly equal to zero and $R(L_2)$ has a nearly direct effect consumption of energy. Therefore, it is very important that the conductivity of shield 14 be maximized. Accurate mathematical models for $R(L_2)$ can only be determined empirically unless very sophisticated computer modeling techniques are used.

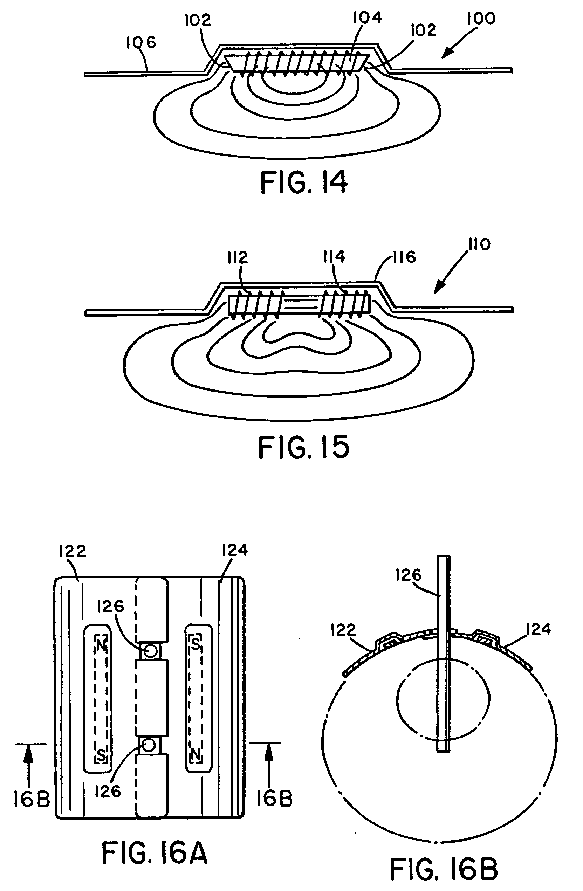

Figure 14 is a technique for enhancing the field distribution of a single dipole shielded dipole transducer. Shielded dipole transducer 100 includes angled end pieces on high permeability bar 104. By angling the end portion away from the shield and towards the surface of the body part under therapy, the magnetic field generated through high permeability bar 104 is directed slightly towards the surface of the body part under therapy. This increases efficiency by directly applying the magnetic field to the body part under therapy and minimizing the field which must be redirected by conductive shield 106.

Figure 15 is another embodiment of the present invention which tailors the magnetic field by separating the coil around high permeability bar 116 into coils 112 and 114. This creates a flux gap which allows the bulging of the flux field in the center of the dipole. This is another method of tailoring the field beneath shielded dipole 110.

In certain therapy regimens for severe non-union problems, electromagnetic therapy may be used in conjunction with invasive techniques for positioning bones in the proper healing position. A shielded transducer particularly adapted for use with such a therapy regimen is shown in Figure 16A. Shielded dipole transducers 122 and 124 are positioned on either side of pins 126. Proper positioning of the pins 126 precludes optimal positioning of a single shielded transducer and thus two transducers are used to allow proper application of the electromagnetic field. Pins 126 are usually composed of steel or other conductive materials which alter the electromagnetic fields in the region by the Lenz effect; however, this effect is minimal. Depending on the desired orientation and resultant efficiency, the coils of shielded dipole transducers 122 and 124 will be connected either in series or parallel.

Figure 16B is a top view of the interleaved relationship of shielded dipole transducers 122, 124 and pins 126. As shown in Figure 16A, the dipole transducers of shielded dipole transducers 122 and 124 provide complimentary magnetic fields as indicated by the N (north) and as S (south) indicators on the dipoles. Alternatively, these dipoles may be wired to generate opposing magnetic fields which may cause desired magnetic field characteristics in certain instances.

Figures 17A and 17B show another embodiment of the present invention. Arcuate shielded dipole 140 includes an arcuate dipole to conform to the body part to allow for transverse application of the shielded dipole transducer 140. In certain fractures, such as hairline fractures, the fracture occurs along the major axis of the bone. Although the precise mechanism for enhanced bone growth is not known, some researchers have suggested that applying a field transverse to the fracture generates the physical mechanism which promotes bone growth.

Figures 18A and 18B show another embodiment of the present invention where the dipole itself is twisted and curved around the body part to provide a perpendicular field to an angled fracture.

Figure 19 is a quadripole shielded transducer including an arcuate dipole 152 and a linear dipole 154, both shielded by conductive shield 156. In certain instances, the complex magnetic fields generated by two dipoles may prove beneficial.

Figure 20 shows another embodiment of the present invention wherein shielded dipole 10 is positioned near high permeability piece 160. High permeability piece 160 may also be positioned diametrically opposed to shielded dipole 10. The positioning of high permeability piece 160 alters the magnetic flux like the presence of a conductor alters the electrical flux in an electrical field. The field altering characteristics of the positioning of high permeability piece 160 can aid in directing the magnetic field towards the desired body portion or modify the field distribution.

Although specific embodiments of the present invention are herein disclosed, they are not to be construed as limiting the scope of the invention. For example, although devices shown for generating magnetic fields from electrical current are coils surrounding high permeability materials any electrical to

7

electromagnetic transducer is useful in conjunction with the present invention and is another embodiment of the present invention. The scope of the invention is only limited by the claims appended hereto.

**Claims**

1. A transducer for converting an electrical signal into an electromagnetic field for application of said electromagnetic field to a damaged body part to promote healing of said body part, said transducer comprising:

a conductive coil, said coil having first and second terminals at opposite ends of said coil, said first and second terminals connected to receive said electrical signals said coil providing an electromagnetic field in response to said electrical signal and said coil providing said electromagnetic field to said damaged body part when placed proximal to said damaged body part; and

a conductive plate adapted to be positioned adjacent to said conductive coil and opposite from said damaged body part, said plate having a major surface adapted to be positioned parallel to said damaged body part and said plate extending laterally beyond said conductive coil in the direction of said major surface.

2. A transducer for converting an electrical signal into an electromagnetic field for application of said electromagnetic field to a damaged body part to promote healing of said body part, said transducer comprising:

a core piece comprising high permeability material;

a conductive coil wrapped around said core piece, said coil having first and second terminals at opposite ends of said coil, said first and second terminals connected to receive said electrical signal, said coil and said core piece providing an electromagnetic field in response to said electrical signal and said coil and core piece providing said electromagnetic field to said damaged body part when placed proximal to said damaged body part; and

a conductive plate adapted to be positioned adjacent to said core piece and conductive coil and opposite from said damaged body part, said plate having a major surface adapted to be positioned parallel to said damaged body part and said plate extending laterally beyond said core piece and conductive coil in the direction of said major surface.

3. A transducer for converting an electrical signal into an electromagnetic field for application of said electromagnetic field to a damaged body part to promote healing of said body part, said transducer comprising:

a plurality of core pieces comprising high permeability material;

a conductive coil wrapped around said core pieces, said coil having first and second terminals at opposite ends of said coil, said first and second terminals connected to receive said electrical signal, said coil and said core pieces providing an electromagnetic field in response to said electrical signal and said coil and core piece providing said electromagnetic field to said damaged body part when placed proximal to said damaged body part; and

a plurality of conductive plates one for each of said core pieces adapted to be positioned adjacent to said core pieces and conductive coil and opposite from said damaged body part, said plates having a major surface adapted to be positioned parallel to said damaged body part and said plates extending beyond said core piece and conductive coil in the direction of said major surface.

4. A transducer for converting an electrical signal into an electromagnetic field for application of said electromagnetic field to a damaged body part to promote healing of said body part, said transducer comprising:

first and second core pieces comprising high permeability material;

a conductive coil wrapped around said first and second core pieces, said coil having first and second terminals at opposite ends of said coil, said first and second terminals connected to receive said electrical signal, said coil and said first and second core pieces providing perpendicular electromagnetic fields in response to said electrical signal and said coil and core piece providing said electromagnetic field to said damaged body part when placed proximal to said damaged body part; and

a conductive plate adapted to be positioned adjacent to said first and second core pieces and conductive coil, and opposite from said damaged body part, said plate having a major surface adapted to be positioned parallel to said damaged body part and said plate extending laterally beyond said first and second core pieces and conductive coil in the direction of said major surface.

**5.** A transducer for converting an electrical signal into an electromagnetic field for application of said electromagnetic field to a damaged body part to promote healing of said body part, said transducer comprising:

a core piece comprising high permeability material said core piece being curved to conform to said damaged body part;

a conductive coil wrapped around said core piece, said coil having first and second terminals at opposite ends of said coil, said first and second terminals connected to receive said electrical signal, said coil and said core piece providing an electromagnetic field in response to said electrical signal and said coil and core piece providing said electromagnetic field to said damaged body part when placed proximal to said damaged body part; and

a conductive plate adapted to be positioned adjacent to said core piece and conductive coil and opposite from said damaged body part, said plate having a major surface adapted to be positioned parallel to said damaged body part and said plate extending beyond said core piece and conductive coil in the direction of said major surface.

**6.** A transducer for converting an electrical signal into an electromagnetic field for application of said electromagnetic field to a damaged body part to promote healing of said body part, said transducer comprising:

a core piece comprising high permeability material;

a conductive coil wrapped around said core piece, said coil having first and second terminals at opposite ends of said coil, said first and second terminals connected to receive said electrical signal, said coil and said core piece providing an electromagnetic field in response to said electrical signal and said coil and core piece providing said electromagnetic field to said damaged body part when placed proximal to said damaged body part;

a shunt piece of high permeability material placed adjacent to said damage body part; and

a conductive plate adapted to be positioned adjacent to said core piece and conductive coil and opposite from said damaged body part, said plate having a major surface adapted to be positioned parallel to said damaged body part and said plate extending beyond said core piece and conductive coil in the direction of said major surface.

**7.** A transducer for converting an electrical signal into an electromagnetic field for application of said electromagnetic field to a damaged body part to promote healing of said body part, said transducer comprising:

a core piece comprising high permeability material;

a plurality of electrically connected conductive coils wrapped around said core piece, said plurality of coils having first and second terminals at opposite ends of said plurality of coils, said first and second terminals connected to receive said electrical signal, said plurality of coils and said core piece providing an electromagnetic field in response to said electrical signal and said plurality of coils and core piece providing said electromagnetic field to said damaged body part when placed proximal to said damaged body part; and

a conductive plate adapted to be positioned adjacent to said core piece and plurality of conductive coils and opposite from said damaged body part, said plate having a major surface adapted to be positioned parallel to said damaged body part and said plate extending beyond said core piece and plurality of conductive coils in the direction of said major surface.

**8.** A transducer as in any of Claims 1-7 wherein said plate includes an indentation adapted for said coil, said major surface of said plate being closer to said damaged body part in the area extending beyond said coil than the area of said major surface not extending beyond said conductive coil.

**9.** A transducer as in Claim 8 wherein said area of said major surface extending beyond said coil is substantially flush with the surface of said coil nearest said damaged body part.

**10.** A transducer as in any of Claims 1-7 where said conductive plate comprises a flexible mesh of conductive wire or a layer of conductive and malleable material affixed to a flexible backing, said layer having a regular pattern of holes formed therein.

FIG. 1

FIG. 2

FIG. 3

FIG. 4

FIG. 5

FIG. 6

FIG. 7

FIG. 8

FIG. 9

FIG. IOA

FIG. IO B

SUMMARIZED AMPLITUDE DATA

MEAN STIFFNESS RATIOS
± STANDARD ERROR

$\Delta t_{PW} = 5$ s

$\Delta t_{BW} = 5$ ms

$f_{BW} = 15$ Hz

FIG. 11

FIG. 12A

FIG. 12B

FIG. 13A

FIG. 13B

14

FIG. 14

FIG. 15

FIG. 16A

FIG. 16B

FIG. 17A

FIG. 17B

FIG. 18A

FIG. 18B

FIG. 19

FIG. 20

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 252 594 (LTI BIOMEDICAL INC) <br> * page 4, line 53 - page 5, line 46; figures 6-8,9 * <br> --- | 1,8,9 | A61N2/02 <br> A61N1/40 |
| X | EP-A-0 076 074 (BENTALL) <br> * page 5, line 18 - page 6, line 20 * <br> --- | 1,10 | |
| A | WO-A-8 809 191 (SHINAGAWA) <br> abstract <br> * figures * <br> ----- | 2-7 | |
| | | | TECHNICAL FIELDS SEARCHED (Int. Cl.5) |
| | | | A61N |

The present search report has been drawn up for all claims

| Place of search | Date of completion of the search | Examiner |
|---|---|---|
| THE HAGUE | 29 OCTOBER 1991 | LEMERCIER D.L.L. |

EPO FORM 1503 03.82 (P0401)